# EUROPEAN PATENT APPLICATION

(11) **EP 4 108 757 A1**
(43) Date of publication of application: **28.12.2022**
(21) Application number: 20920348.8
(22) Date of filing: 21.02.2020
(51) Int. Cl.: C12M 1/00, C12N 1/00, F04C 5/00

(54) **SOLUTION CONVEYANCE APPARATUS**

(71) Applicant: I Peace, Inc., Palo Alto, CA 94303 (US); FANUC CORPORATION, Oshino-mura Minamitsuru-gun Yamanashi 401-0597 (JP)
(72) Inventor: TANABE, Koji, Palo Alto, California 94303 (US); HIRAIDE, Ryoji, Kyoto-shi, Kyoto 606-8414 (JP); BAN, Kazunori, Minamitsuru-gun, Yamanashi 401-0597 (JP); KINOSHITA, Satoshi, Minamitsuru-gun, Yamanashi 401-0597 (JP)
(74) Representative: MacLean, Martin Robert
(86) International application number: PCT/JP2020/007103
(87) International publication number: WO 2021/166227

(57) **Abstract**

An solution transfer device comprises a pump 60 and a substrate 70. The pump 60 comprises a tube 1 for transferring a solution; tube rotors 21A, 21B, 21C, which contact the tube 1; and a driver 10 for transferring a solution within the tube 1 by rotating the tube rotors 21A, 21B, 21C without contacting the tube rotors 21A, 21B, 21C. The substrate 70 is provided with a solution-transferring flow path that is connected to the tube 1 of the pump 60.

## Description

### Technical Field

The present invention relates to a solution transfer device.

### Background Art

Pumps are used to move fluids, which may be a liquid or a gas. Contamination of the fluid may occur when the pump is in direct contact with the fluid being transported. Therefore, when contamination of the fluid is undesirable, a tube pump which can transport a fluid within a tube without direct contact with the fluid is used(refer, for example, to Patent Documents 1 and 2). Tube pumps comprise a rotor that rotates while compressing the tube. The compressed region of the tube undergoes a reduction in cross-sectional area. Since the compressed region of the tube moves in accordance with the rotation of the rotor, the fluid within the tube is transferred in the direction of rotation of the rotor.

### Citation List

### Patent Document

Patent Document 1: Japanese Patent No. 4035119
Patent Document 2: Japanese Patent No. 5824685

### Summary

### Technical Problem

An object of the present invention is to provide a solution transfer device that can transfer a solution.

### Solution to Problem

According to an aspect of the present invention, there is provided a solution transfer device comprising: a pump comprises a tube for transferring a solution, a tube rotor that contacts the tube, and a driver that rotates the tube rotor without contacting the tube rotor, in order to transfer the solution within the tube; and a substrate provided with a flow path for transferring the solution, wherein the flow path is connected to the tube of the pump.

The flow path in the aforementioned solution transfer device may be provided in the interior of the substrate.

The solution in the aforementioned solution transfer device may be a solution containing a cell.

The solution in the aforementioned solution transfer device may be a solution that contains a factor to be introduced into cells.

The solution in the aforementioned solution transfer device may be a solution containing a reagent.

The solution in the aforementioned solution transfer device may be a solution containing a culture medium.

A cell treatment section that treats cells and is connected to the flow path may be provided at the substrate in the aforementioned solution transfer device.

The cell treatment section in the aforementioned solution transfer device may be a purification section for purifying cells.

The cell treatment section in the aforementioned solution transfer device may be a factor introduction section for introducing a factor to cells.

The cell treatment section in the aforementioned solution transfer device may be a culture section for culturing cells.

The culture section in the aforementioned solution transfer device may engage in the initialization culture of cells.

The culture section in the aforementioned solution transfer device may engage in the expansion culture of cells.

The cell treatment section in the aforementioned solution transfer device may be a differentiation induction section for inducing the differentiation of cells.

The cell treatment section in the aforementioned solution transfer device may be disposed in the interior of the substrate.

The cell treatment section in the aforementioned solution transfer device may be disposed to the outside of the substrate.

The cell treatment section in the aforementioned solution transfer device may project out from a side surface of the substrate.

The tube and the tube rotor of the pump may be located outside of the substrate in the aforementioned solution transfer device.

The tube and the tube rotor of the pump may be located in the substrate in the aforementioned solution transfer device.

The driver may be located outside of the substrate in the aforementioned solution transfer device.

In the aforementioned solution transfer device, the pump may further comprise a magnet that is connected to the tube rotor; and the driver may cause rotation of the tube rotor by magnetic force using the magnet.

In the aforementioned solution transfer device, the substrate may be provided with a first substrate section having a first contact surface and with a second substrate section having a second contact surface that contacts the first contact surface, and the flow path may be provided in at least one of the first contact surface and the second contact surface.

In the aforementioned solution transfer device, the substrate may be provided with a first substrate section having a first contact surface and with a second substrate section having a second contact surface that contacts the first contact surface, and a cell treatment section may be provided in at least one of the first contact surface and the second contact surface.

The pump in the aforementioned solution transfer device may further comprise a case that houses the tube and the tube rotor.

The driver in the aforementioned solution transfer device may rotate the tube rotor, from outside the case, without contacting the tube rotor.

The case in the aforementioned solution transfer device may be closed.

The case in the aforementioned solution transfer device may be fluid impermeable.

The case in the aforementioned solution transfer device may be liquid impermeable.

In the aforementioned solution transfer device, an inlet connector and an outlet connector may be provided in the case, and one end of the tube may be connected to the inlet connector and the other end of the tube may be connected to the outlet connector.

A depression that houses the tube and the tube rotor may be provided inside the case in the aforementioned solution transfer device.

A magnet may be provided in the tube rotor in the aforementioned solution transfer device.

In the aforementioned solution transfer device, the pump may further comprise a transmission rotor connected to the tube rotor and a magnet may be provided in the transmission rotor.

In the aforementioned solution transfer device, the pump may further comprise an internal drive rotor that connects the tube rotor and the transmission rotor.

A depression that houses the transmission rotor may be provided inside the case in the aforementioned solution transfer device.

The pump in the aforementioned solution transfer device may further comprise a light reflector connected to a part of the tube rotor.

The pump in the aforementioned solution transfer device may further comprise a light source that irradiates light on the light reflector from outside the case, and with a light-receiver that receives the light reflected from the light reflector.

The pump in the aforementioned solution transfer device may further comprise a rotation rate calculator that calculates the rotation rate of the tube rotor based on the reflected light received by the light-receiver.

The aforementioned solution transfer device may be provided, at an outer wall of the case, with a housing that houses the light source and the light-receiver.

The case and the driver may be detachable in the aforementioned solution transfer device.

Also according to an aspect of the present invention, there is provided a solution transfer device comprising: a pump comprising a tube for transferring solution disposed along at least a portion of a side surface of a circle-shape depression, a tube rotor disposed to be contactable with the tube at within the depression, and an internal drive rotor that contacts the tube rotor, wherein, in the case where the internal drive rotor rotates, the tube rotor is rotated by frictional force between the internal drive rotor and the tube rotor; and a substrate provided with a flow path for transferring the solution, wherein the flow path is connected to the tube of the pump.

The flow path in the aforementioned solution transfer device may be provided in the interior of the substrate.

The solution in the aforementioned solution transfer device may be a solution containing a cell.

The solution in the aforementioned solution transfer device may be a solution that contains a factor to be introduced into cells.

The solution in the aforementioned solution transfer device may be a solution containing a reagent.

The solution in the aforementioned solution transfer device may be a solution containing a culture medium.

A cell treatment section that treats cells and is connected to the flow path may be provided at the substrate in the aforementioned solution transfer device.

The cell treatment section in the aforementioned solution transfer device may be a purification section for purifying cells.

The cell treatment section in the aforementioned solution transfer device may be a factor introduction section for introducing a factor to cells.

The cell treatment section in the aforementioned solution transfer device may be a culture section for culturing cells.

The culture section in the aforementioned solution transfer device may engage in the initialization culture of cells.

The culture section in the aforementioned solution transfer device may engage in the expansion culture of cells.

The cell treatment section in the aforementioned solution transfer device may be a differentiation induction section for inducing the differentiation of cells.

The cell treatment section in the aforementioned solution transfer device may be disposed in the interior of the substrate.

The cell treatment section in the aforementioned solution transfer device may be disposed to the outside of the substrate.

The cell treatment section in the aforementioned solution transfer device may project out from a side surface of the substrate.

The depression may be provided in the substrate in the aforementioned solution transfer device.

The pump in the aforementioned solution transfer device may further comprise a case in which the depression is provided.

The case of the pump may be located to the outside of the substrate in the aforementioned solution transfer device.

The case of the pump may be located in the substrate in the aforementioned solution transfer device.

The pump in the aforementioned solution transfer device may further comprise a driver for transferring the solution in the tube by rotating the internal drive rotor without contacting the internal drive rotor.

The driver in the aforementioned solution transfer device may be located outside of the substrate.

The substrate in the aforementioned solution transfer device may be provided with a first substrate section having a first contact surface and with a second substrate section having a second contact surface that contacts the first contact surface, and the flow path may be provided in at least one of the first contact surface and the second contact surface.

The substrate in the aforementioned solution transfer device may be provided with a first substrate section having a first contact surface and with a second substrate section having a second contact surface that contacts the first contact surface, and the cell treatment section may be provided in at least one of the first contact surface and the second contact surface.

The tube rotor in the aforementioned solution transfer device may rotate around the center of the tube rotor and may rotate within the depression centered on the internal drive rotor.

The radius of the tube rotor in the aforementioned solution transfer device may be longer than the radius of the internal drive rotor.

Of the side surface of the depression, the tube rotor in the aforementioned solution transfer device may contact the side surface in the region of the side surface where the tube is not disposed.

In the aforementioned solution transfer device, unevenness may be provided in the surface of the internal drive rotor that contacts the tube rotor.

### Advantageous Effects of Invention

The present invention can thus provide a solution transfer device that can transfer a solution.

### Brief Description of Drawings

Fig. 1 is a schematic perspective diagram of a solution transfer device according to a first embodiment.
Fig. 2 is a schematic perspective diagram of the solution transfer device according to the first embodiment.
Fig. 3 is a schematic side view of the solution transfer device according to the first embodiment.
Fig. 4 is a schematic perspective diagram of the pump according to the first embodiment.
Fig. 5 is a schematic exploded perspective view of the pump according to the first embodiment.
Fig. 6 is a schematic perspective diagram of the pump according to the first embodiment.
Fig. 7 is a schematic cross-sectional diagram of the pump according to the first embodiment.
Fig. 8 is a schematic exploded perspective view of the solution transfer device according to the first embodiment.
Fig. 9 is a schematic perspective diagram of a solution transfer device according to a second embodiment.
Fig. 10 is a schematic side view of the solution transfer device according to the second embodiment.
Fig. 11 is a schematic rear view of the solution transfer device according to the second embodiment.
Fig. 12 is a schematic exploded perspective view of the solution transfer device according to the second embodiment.
Fig. 13 is a schematic perspective diagram of a pump according to a third embodiment.
Fig. 14 is a schematic perspective diagram of the pump according to the third embodiment.
Fig. 15 is a schematic rear view of the pump according to the third embodiment.
Fig. 16 is a schematic rear view of a pump according to a fourth embodiment.
Fig. 17 is a schematic rear view of the pump according to the fourth embodiment.
Fig. 18 is a schematic rear view of the pump according to the fourth embodiment.
Fig. 19 is a schematic rear view of the pump according to the fourth embodiment.
Fig. 20 is a schematic perspective diagram of a solution transfer device according to a fifth embodiment.
Fig. 21 is a schematic perspective diagram of a solution transfer device according to a sixth embodiment.

### Description of Embodiments

Embodiments of the present invention are described hereinbelow. The same or similar reference signs are assigned to the same or similar elements in the description of the figures that follows. However, the figures are schematic diagrams. Thus, the specific dimensions and so forth should be evaluated in light of the following description. In addition, the respective figures naturally contain elements having different dimensional relationships and ratios therebetween.

### (First Embodiment)

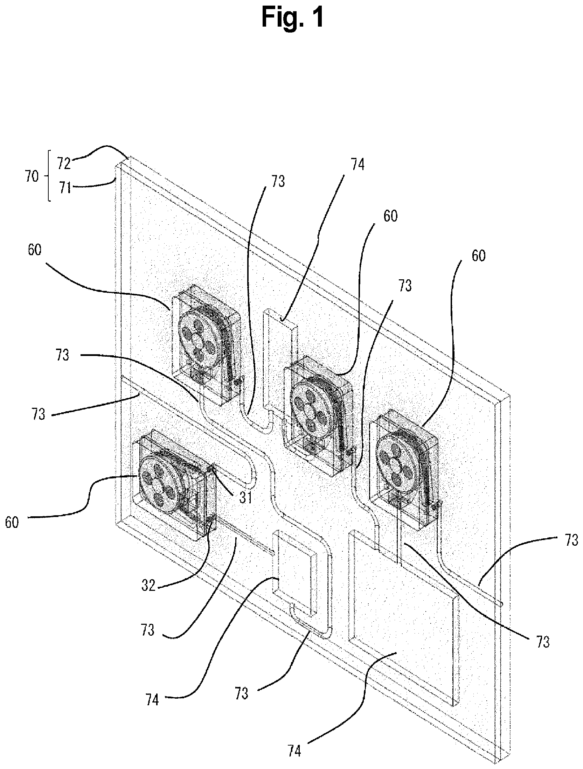

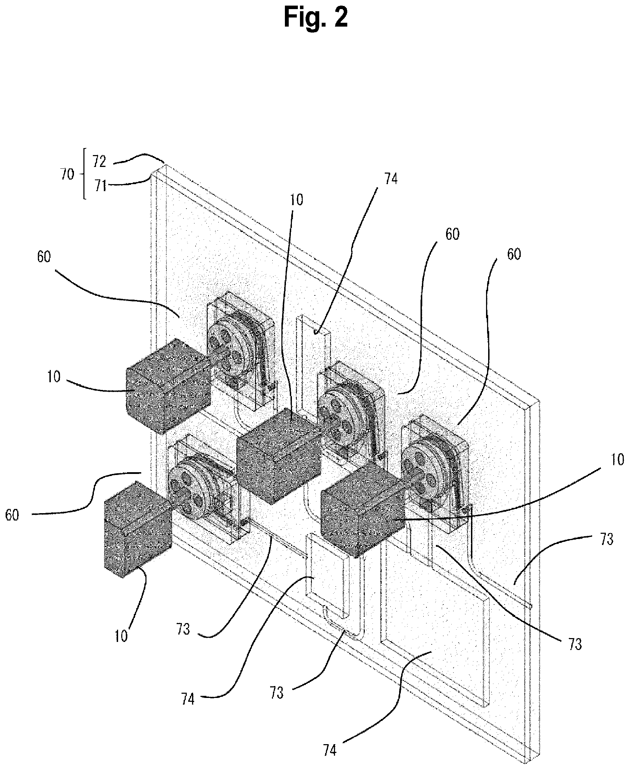

As shown in Figs. 1, 2, and 3, the solution transfer device according to a first embodiment comprises a pump 60 and a substrate 70. The solution transfer device may comprise a plurality of the pumps 60. The solution transfer device may comprise a plurality of pumps that are not shown, in addition to the pumps 60 that are shown.

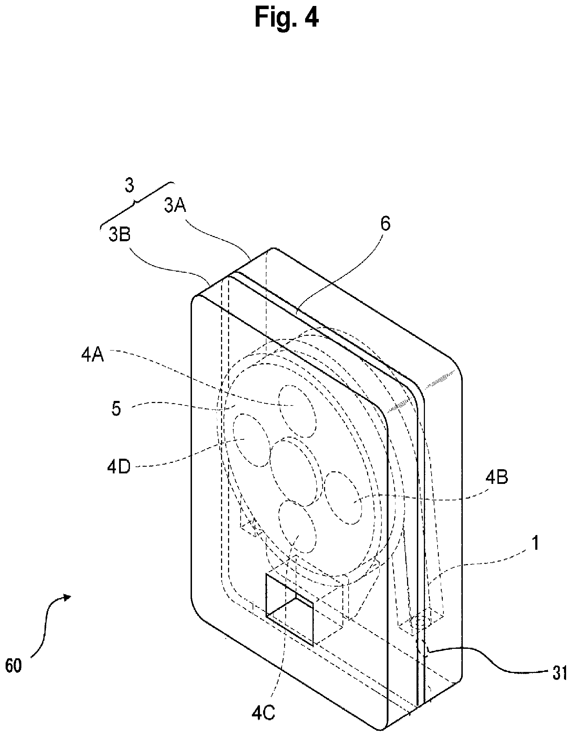

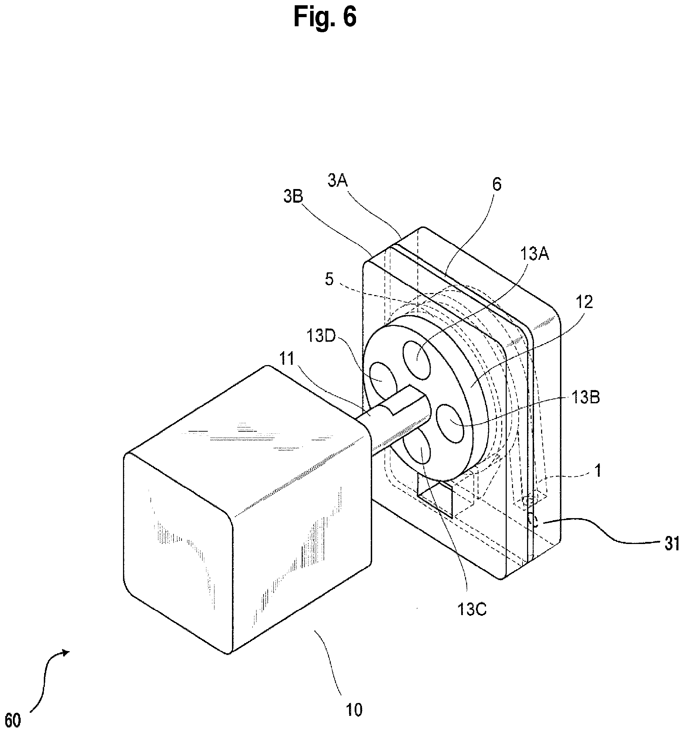

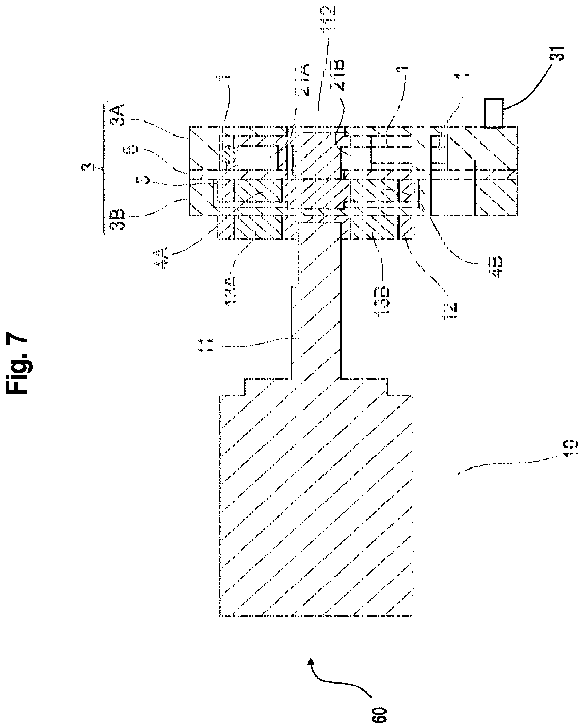

The pump 60 is a tube pump, and, as shown in Figs. 4 to 7, it comprises a tube 1 for transferring a solution; tube rotors 21A, 21B, 21C, which are in contact with the tube 1; and a driver 10 for transferring a solution within the tube 1 by causing the rotation of the tube rotors 21A, 21B, 21C without contacting the tube rotors 21A, 21B, 21C. The solution, for example, contains cells.

The cells can be exemplified by somatic cells, but are not particularly limited. The cells can be exemplified by fibroblasts, nerve cells, retinal epithelial cells, hepatocytes, β-cells, renal cells, blood cells, dental pulp stem cells, keratinocytes, hair papilla cells, oral epithelial cells, megakaryocytes, T cells, NK cells, NKT cells, cartilage cells, myocardial cells, muscle cells, vascular cells, epithelial cells, factor-transduced cells, reprogrammed cells, and stem cells, although there is no particular limitation to these. The stem cells can be exemplified by mesenchymal stem cells, somatic stem progenitor cells, pluripotent stem cells, ES cells, and iPS cells.

The pump 60 may further comprise a case 3 that houses the tube 1 and the tube rotors 21A, 21B, 21C. The driver 10 may rotate, from outside the case 3, the tube rotors 21A, 21B, 21C without contacting the tube rotors 21A, 21B, 21C.

The tube 1, for example, has flexibility. The material of the tube 1 is selected, for example, so the interior of the tube 1 does not undergo exchange across the wall of the tube 1 with, for example, gases, viruses, microorganisms, impurities, and so forth, from the outside. At least a portion of the tube 1 is disposed within the case 3 so as to form a circle-shape portion.

There are no particular limitations on the number of tube rotors 21A, 21B, 21C. Each of the tube rotors 21A, 21B, 21C comes into contact with the inner circumference of the circle-shape tube 1 and applies pressure to the tube 1. Each of the tube rotors 21A, 21B, 21C is rotatable with respect to the center of the circle-shape portion formed by the tube 1. In addition, each of the tube rotors 21A, 21B, 21C is rotatable within the case 3 while partially compressing the tube 1. The solution in the tube 1 is transferred, in accordance with the direction of rotation of the tube rotors 21A, 21B, 21C, by the rotation of each of the tube rotors 21A, 21B, 21C while same is partially compressing the tube 1. Because the tube rotors 21A, 21B, 21C are not in contact with the interior of the tube 1, the solution within the tube 1 can be transferred within the tube 1 without coming into contact with the tube rotors 21A, 21B, 21C.

The case 3, for example, can be separable into a case 3A and a case 3B. The case 3A and the case 3B, for example, may be engaged with each other. The material of the case 3, for example, is not fluid permeable. For example, a depression 33 that houses the tube 1 and the tube rotors 21A, 21B, 21C is provided inside the case 3. In addition, for example, an inlet connector 31 and an outlet connector 32, as shown in Fig. 1, are provided in the case 3. One end of the tube 1 is connected to the inlet connector 31, and the other end of the tube 1 is connected to the outlet connector 32. The inlet connector 31 and the outlet connector 32 may be executed as a single article with the case 3. Alternatively, the inlet connector 31 and outlet connector 32 may be detachable from the case 3. In this case, the inlet connector 31 and the outlet connector 32 may be disposed in the case 3 via an interposed sealing member, e.g., an O-ring.

The interior of the case 3 is closed off from the outside in the case where the inlet connector 31 and the outlet connector 32 are connected to the two ends of the tube 1 and the case 3A and the case 3B shown in Figs. 4 to 7 are engaged. The interior of the closed case 3 may be under a vacuum. The interior of the closed case 3 may be filled with an inert gas, e.g., nitrogen or argon. The interior of the closed case 3 may be filled with a liquid or a gel. In the case where the case 3 is closed, even if a fluid is present within the case 3, the fluid within the case 3 cannot escape to the outside. In addition, in the case where the case 3 is closed, fluid on the outside of the case 3 cannot enter into the interior of the case 3. As a consequence, the interior of the case 3 cannot exchange, e.g., gases, viruses, microorganisms, impurities, and so forth, with the outside.

The pump 60 may comprise magnets 4A, 4B, 4C, 4D connected to the tube rotors 21A, 21B, 21C. The number of magnets may be freely selected. The magnets can be exemplified by permanent magnets and electromagnets. The driver 10 disposed on the outside of the case 3A may rotate the tube rotors 21A, 21B, 21C within the case 3 by magnetic force using the interposed magnets 4A, 4B, 4C, 4D. The magnets may be provided in the tube rotors 21A, 21B, 21C. Alternatively, the pump 60 may comprise a transmission rotor 5 connected to the tube rotors 21A, 21B, 21C and the magnets 4A, 4B, 4C, 4D may be disposed in the transmission rotor 5. For example, the magnets 4A, 4B, 4C, 4D are located at equal intervals on the circumference in the transmission rotor 5. For example, the magnets 4A, 4B, 4C, 4D may be inserted in openings provided in the transmission rotor 5. The transmission rotor 5 may be constructed such that the magnets 4A, 4B, 4C, 4D will not escape from the openings provided in the transmission rotor 5. A drive rotor 112 is connected to the center of the transmission rotor 5. The drive rotor 112 is in contact with the tube rotors 21A, 21B, 21C. In the case where the driver 10 rotates the transmission rotor 5 by magnetic force, the drive rotor 112 rotates accompanying the rotation of the transmission rotor 5 and the tube rotors 21A, 21B, 21C rotate accompanying the rotation of the drive rotor 112.

The tube 1 and the tube rotors 21A, 21B, 21C may be disposed within the case 3A. In this instance, the case 3A is provided with a depression 33 that houses or accommodates the tube 1 and the tube rotors 21A, 21B, 21C. The transmission rotor 5 may be disposed within the case 3B. A depression 34 that houses or accommodates the transmission rotor 5 may be provided in the case 3B. A shaft holder 6, which holds the shaft-equipped drive rotor 112, may be disposed within the case 3. The shaft holder 6, for example, is placed between the tube rotors 21A, 21B, 21C and the transmission rotor 5. The shaft holder 6 holds the drive rotor 112, and as a result the tube rotors 21A, 21B, 21C, drive rotor 112, and transmission rotor 5 are held in prescribed positions within the case 3. However, for example, the shaft holder 6 may not be present as the depression 33 provided in the case 3A is capable of holding the tube rotors 21A, 21B, 21C and the depression 34 provided in the case 3B is capable of holding the transmission rotor 5.

The driver 10 comprises a drive shaft 11 and an external drive rotor 12, which is connected at its center to the drive shaft 11. Magnets 13A, 13B, 13C, 13D are provided in the external drive rotor 12. The magnets 13A, 13B, 13C, 13D, for example, are located at equal intervals on the circumference of the external drive rotor 12. The number of magnets provided in the external drive rotor 12 may be freely selected, but the number of magnets provided in the external drive rotor 12 is preferably the same as the number of magnets connected to the tube rotors 21A, 21B, 21C. In addition, preferably the magnets provided in the external drive rotor 12 and the magnets connected to the tube rotors 21A, 21B, 21C are arranged with the same spacing on circumferences having the same size. The magnets 13A, 13B, 13C, 13D provided in the external drive rotor 12 and the magnets 4A, 4B, 4C, 4D connected to the tube rotors 21A, 21B, 21C attract each other.

The driver 10 is located outside the case 3. The external drive rotor 12 of the driver 10 is disposed such that the magnets 13A, 13B, 13C, 13D of the external drive rotor 12 face the magnets 4A, 4B, 4C, 4D connected to the tube rotors 21A, 21B, 21C, with the case 3 sandwiched therebetween. Because the magnets 13A, 13B, 13C, 13D provided in the external drive rotor 12 and the magnets 4A, 4B, 4C, 4D connected to the tube rotors 21A, 21B, 21C attract each other, the tube rotors 21A, 21B, 21C also rotate in the case where the external drive rotor 12 rotates.

The substrate 70 shown in Fig. 1 can have a structure that enables the interior to be closed off from the outside air. The material of the substrate 70 can be exemplified by resins and glasses. The substrate 70 may be transparent.

The interior of the substrate 70 shown in Fig. 1 is provided with a flow path 73 that is connected to the tube 1 of the pump 60 and is for transporting the solution containing a cell. A plurality of flow paths 73 may be provided in the substrate 70. A plurality of flow paths that are not shown may be provided in the substrate 70 in addition to the flow paths 73 that are shown. For example, the pump 60 suctions the solution containing the cell from the flow path 73 provided in the substrate 70. In addition, the pump 60 transports the solution containing the cell to the flow path 73 provided in the substrate 70.

The interior of the substrate 70 may be provided with a cell treatment section 74, which is connected to the flow path 73 and functions to treat cells. A plurality of cell treatment sections 74 may be provided in the substrate 70. A plurality of cell treatment sections that are not shown may be provided in the substrate 70 in addition to the cell treatment sections 74 that are shown. The pump 60, for example, suctions the solution containing the cell from the cell treatment section 74 through the flow path 73 provided in the substrate 70. In addition, the pump 60 transports the solution containing the cell to the cell treatment section 74 through the flow path 73 provided in the substrate 70. In the case where a plurality of cell treatment sections 74 are provided in the substrate 70, cells are transported from an upstream cell treatment section 74 to a downstream cell treatment section 74 through the flow path 73 and the pump 60.

The closed-off space comprising the interior of the flow path 73 and the cell treatment section 74 can be configured such that there is no exchange with gases, viruses, microorganisms, impurities, and so forth, from the outside.

Cell treatments that are different from each other may be performed in the plurality of cell treatment sections 74. The same cell treatment may be performed in a plurality of cell treatment sections 74. A plurality of cell treatments may be performed in a single cell treatment section 74.

The cell treatment section 74 may be a purification section for the purification of cells. A specific cell may be purified from a plurality of cells in the cell treatment section 74. In this case, for example, a substance for cell purification, e.g., a reagent, is introduced into the cell treatment section 74. A substance for cell purification may be transported into the cell treatment section 74 through a pump and flow path that are not shown. The substance for cell purification, for example, causes the sedimentation of cells other than the purification target. Alternatively, the substance for cell purification, for example, causes the lysis of cells other than the purification target. For example, mononuclear cells are purified from blood in the cell treatment section 74. In this case, for example, an erythrocyte sedimentation agent or erythrocyte removal agent for the removal of erythrocytes is introduced into the cell treatment section 74. Alternatively, the purification of mononuclear cells from blood may be carried out by introducing a mononuclear cell purification solution into the cell treatment section 74.

In the case where the cell treatment section 74 is a purification section for cell purification, a cell treatment member may be disposed in the cell treatment section 74. A filter is an example of a cell treatment member. The filter can be exemplified by hollow fiber membranes and flat membranes.

The cell treatment section 74 may be a factor introduction section for the introduction of a factor into cells. In this case, for example, the factor to be introduced into the cells is input into the cell treatment section 74. The factor for introduction into cells may be transported into the cell treatment section 74 through a flow path and pump that are not shown. The cells for factor introduction may be cells that have been purified in a separate cell treatment section 74. For example, cells may be transported, through the flow path 73 and the pump 60, from a cell treatment section 74 for cell purification to a cell treatment section 74 for the introduction of a factor into the cells.

The cells for factor introduction may be attached in the interior of the cell treatment section 74. Alternatively, the cells for factor introduction may be suspension cultured (three-dimensional culture) in the cell treatment section 74. In the case where the cells for factor introduction are attached in the interior of the cell treatment section 74, after the introduction of the factor into the cells, the cells may be detached from the interior of the cell treatment section 74 using a dissociation agent. The cells may be cultured within hollow fibers placed within the cell treatment section 74.

The factor may be a nucleic acid, e.g., DNA, RNA, oligonucleotides, and so forth, or may be a protein, or may be a compound, or may be a virus. The DNA may be plasmid DNA. The RNA may be mRNA, siRNA, or miRNA. The RNA may be a modified RNA or may be an unmodified RNA. The nucleic acid may be incorporated in a vector. The vector can be exemplified by plasmids, retroviruses, lentiviruses, adenoviruses, adeno-associated viruses, and Sendai viruses. The protein may be a nuclease protein, e.g., Cas9 protein. The virus may be a lentivirus. The factor may be an induction factor that induces a cell in a first state into a cell in a second state. The factor may be a hormone, growth factor, or low-molecular-weight compound.

In the present disclosure, induction refers to, for example, reprogramming, initialization, transformation, transdifferentiation or lineage reprogramming, differentiation induction, and cell fate reprogramming. Factors that induce cells other than pluripotent stem cells to pluripotent stem cells are referred to as reprogramming factors. Reprogramming factors include, for example, OCT3/4, SOX2, KLF4, and c-MYC. Factors that induce stem cells to differentiated cells are referred to as differentiation induction factors.

Examples of factors that induce cells to nervous system cells are the ASCL family, DLX family, MYT family, NeuroD family, SOX family, and NGN family. The ASCL family can be exemplified by ASCL1. The DLX family can be exemplified by DLX2. The MYT family can be exemplified by MYT1L. The NGN family can be exemplified by NGN2. The nervous system cells can be exemplified by neurons, neural stem cells, and neural progenitor cells. The neurons can be exemplified by inhibitory neurons, excitatory neurons, dopamine-producing neurons, cranial nerves, mediated nerves, and optic nerves. Alternatively, the nervous system cells may be motor neurons, oligodendrocyte progenitor cells, astrocytes, oligodendrocytes, and so forth.

Factors that induce cells to cardiomyocytes can be exemplified by the GATA family, MEF family, TBX family, MYOCD family, MESP family, and miR-133 family. The GATA family can be exemplified by GATA4A. The MEF family can be exemplified by MEF2C. The TBX family can be exemplified by TBX5. The MESP family can be exemplified by MESP1.

The cell treatment section 74 may be a culture section for carrying out cell culture. In this case, for example, a culture medium is introduced into the cell treatment section 74. The culture medium may be introduced into the cell treatment section 74 through a pump and flow path that are not shown. The cells to be cultured may be cells into which a factor has been introduced in a separate cell treatment section 74. The cells may be transported, through the flow path 73 and the pump 60, from the cell treatment section 74 for introducing a factor into the cells, to the cell treatment section 74 for culturing the cells. However, a factor may be introduced into cells and the factor-transduced cells may be cultured in one and the same cell treatment section 74.

The cells to be cultured may be attached in the interior of the cell treatment section 74. Alternatively, the cells to be cultured may be suspension cultured (three-dimensional culture) in the cell treatment section 74. In the casa where the cells to be cultured are attached in the interior of the cell treatment section 74, after cell culture the cells may be detached from the interior of the cell treatment section 74 using a dissociation agent. The cells may be cultured in hollow fibers disposed in the cell treatment section 74. A semipermeable membrane may be disposed in the cell treatment section 74 and cells may be cultured within compartments provided by partitioning of the cell treatment section 74 by the semipermeable membrane. The exchange of components of the culture medium and cell waste products may be carried out across the semipermeable membrane.

The cell treatment section 74 may be an initialization culture section for carrying out the initialization culture of cells. In this case, for example, an initialization culture medium is introduced into the cell treatment section 74. The initialization culture medium may be transported into the cell treatment section 74 through a flow path and pump that are not shown. The cells to be initialization cultured may be cells into which a factor has been introduced in a separate cell treatment section 74. Cells may be transported from the cell treatment section 74 for introducing a factor into the cells, through the flow path 73 and a pump 60, into the cell treatment section 74 for the initialization culture of the cells. However, a factor may be introduced into cells and the factor-transduced cells may be initialization cultured in one and the same cell treatment section 74.

The cells to be initialization cultured may be attached within the cell treatment section 74. Alternatively, the cells to be initialization cultured may be suspension cultured (three-dimensional culture) in the cell treatment section 74. In the case where the cells to be initialization cultured are attached within the cell treatment section 74, after the initialization culture of the cells, the cells may be detached from the interior of the cell treatment section 74 using a dissociation agent.

The cell treatment section 74 may be an expansion culture section for carrying out an expansion culture in order to cause cell proliferation. In this case, for example, an expansion culture medium is introduced into the cell treatment section 74. The expansion culture medium may be transported into the cell treatment section 74 through a pump and flow path that are not shown. The cells to be expansion cultured may be cells that have been initialization cultured in a separate cell treatment section 74. The cells may be transported from the cell treatment section 74 for initialization culture of the cells, through the flow path 73 and the pump 60, to the cell treatment section 74 for expansion culture of the cells. However, cell initialization culture and additional cell expansion culture may be performed in one and the same cell treatment section 74. Introduction of a factor into cells, initialization culture of the factor-transduced cells, and additional expansion culture of the cells may be performed in one and the same cell treatment section 74.

The cells to be expansion cultured may be attached in the interior of the cell treatment section 74. Alternatively, the cells to be expansion cultured may be suspension cultured (three-dimensional culture) in the cell treatment section 74. In the case where the cells to be expansion cultured are attached in the interior of the cell treatment section 74, after the cells have been expansion cultured, the cells may be detached from within the cell treatment section 74 using a dissociation agent.

The cell treatment section 74 may be a differentiation induction section for inducing cell differentiation. In this case, for example, a differentiation induction factor is introduced into the cell treatment section 74. The differentiation induction factor may be transported into the cell treatment section 74 through a flow path and pump that are not shown. Cells in a first state may be induced to differentiate into cells in a second state in the differentiation induction section. For example, stem cells may be induced to differentiate into somatic cells other than stem cells in the differentiation induction section. Alternatively, somatic cells other than stem cells may be induced to differentiate into different somatic cells other than stem cells in the differentiation induction section. Somatic cells other than stem cells may be directly reprogrammed to different somatic cells other than stem cells.

The cells for which differentiation is to be induced may be attached within the cell treatment section 74. Alternatively, the cells for which differentiation is to be induced may be suspension cultured (three-dimensional culture) within the cell treatment section 74. In the case where the cells for which differentiation is to be induced are attached in the interior of the cell treatment section 74, the induction of cell differentiation may be followed by detachment of the cells from the interior of the cell treatment section 74 using a dissociation agent.

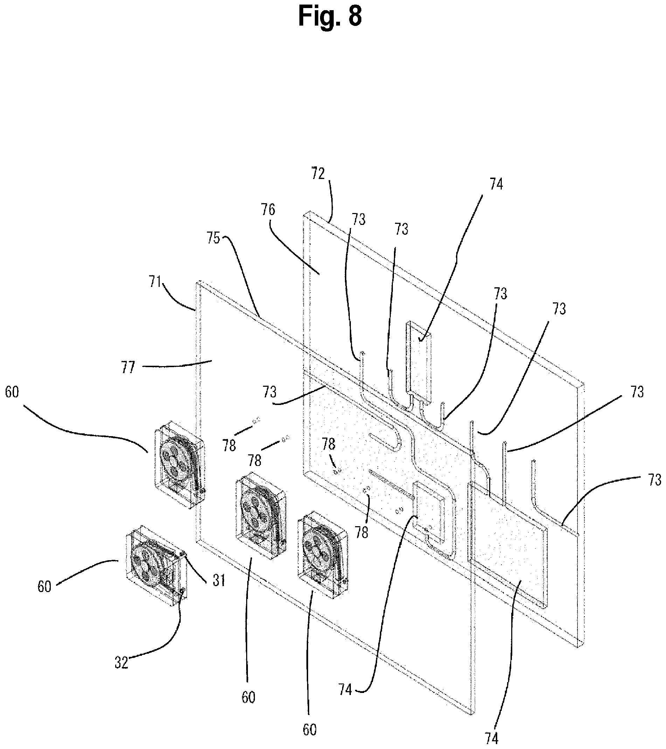

As shown in Fig. 8, the substrate 70 may comprise a first substrate section 71, which has a first contact surface 75, and a second substrate section 72, which has a second contact surface 76 that contacts the first contact surface 75. For example, the first substrate section 71 and the second substrate section 72 are stuck to each other so that the first contact surface 75 is in contact with the second contact surface 76. The first substrate section 71 may be stuck to the second substrate section 72 by a heat welding method or a pressure welding method or an ultrasound welding method. The first substrate section 71 may be bonded to the second substrate section 72 using an adhesive.

A flow path 73 may be provided in at least one of the first contact surface 75 and the second contact surface 76. The flow path 73 may be formed from a depression that is provided in at least one of the first contact surface 75 and the second contact surface 76. The flow path 73 is formed in the interior of the substrate 70 by sticking the first substrate section 71 to the second substrate section 72, for which a depression is provided in at least one of the first contact surface 75 and the second contact surface 76. In the case where a depression for forming the flow path 73 is provided in at least one of the first contact surface 75 and the second contact surface 76, the surface where the depression is not provided may be flat. In the case where depressions for forming the flow path 73 are provided in both the first contact surface 75 and the second contact surface 76, this disposition may be configured such that the location of the depression provided in the first contact surface 75 matches the location of the depression provided in the second contact surface 76 in the case where the first substrate section 71 is stuck with the second substrate section 72.

The cell treatment section 74 may be provided in at least one of the first contact surface 75 and the second contact surface 76. The cell treatment section 74 may be formed from a depression that is provided in at least one of the first contact surface 75 and the second contact surface 76. The cell treatment section 74 is formed within the substrate 70 by sticking the first substrate section 71 to the second substrate section 72, for which a depression is provided in at least one of the first contact surface 75 and the second contact surface 76. In the case where either of the first contact surface 75 and the second contact surface 76 is provided with a depression for forming the cell treatment section 74, the surface where the depression is not provided may be flat. In the case where depressions for forming a cell treatment section 74 are provided in both the first contact surface 75 and the second contact surface 76, this disposition may be configured such that the location of the depression provided in the first contact surface 75 matches the location of the depression provided in the second contact surface 76 in the case where the first substrate section 71 is stuck with the second substrate section 72.

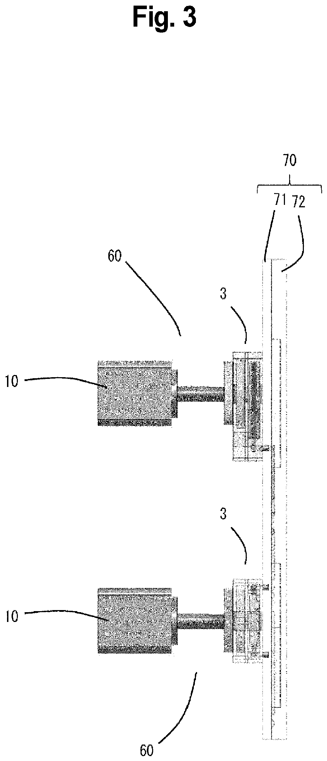

The case 3 that houses the tube 1 and the tube rotors 21A, 21B, 21C of the pump 60 may be located outside the substrate 70. As shown in Fig. 3, the tube rotors 21A, 21B, 21C are contactlessly rotated from outside the case 3. The driver 10 for transferring a solution within the tube 1 may be disposed outside the substrate 70.

For example, as shown in Fig. 8, the case 3 of the pump 60 may be located on an outside surface 77 that resides opposite to the first contact surface 75 of the first substrate section 71. For example, there may be disposed, in the first substrate section 71, a hole 78 for connecting the tube 1 of the pump 60 with a depression that forms the flow path 73 or the cell treatment section 74 and is provided in at least either of the first substrate section 71 and the second substrate section 72. An inlet connector 31 and an outlet connector 32, which are provided in the case 3 of the pump 60, may be inserted into the holes 78 that are provided in the first substrate section 71.

With the pump 60 according to the first embodiment, the tube rotors 21A, 21B, 21C in the case 3-shielded interior can be contactlessly rotated by the driver 10 that is located outside the case 3. As a consequence, even if the tube 1 were to be damaged, scattering or dispersion of the substance in the tube 1 to the outside of the case 3 can be prevented. The entry of substances outside the case 3 into the tube 1, and thus the occurrence of contamination of the fluid within the tube 1, can also be prevented. As a consequence, the introduction of viruses and bacteria into the solution in the tube 1 can be prevented in the case where the solution containing the cell is transported by the pump 60. In addition, in the case where, for example, the solution containing the cell that should be held at a constant pH is transported by the pump 60, the introduction of an acidic or alkaline fluid into the tube 1, and thus changes in the pH of the solution in the tube 1, can be prevented.

### (Second Embodiment)

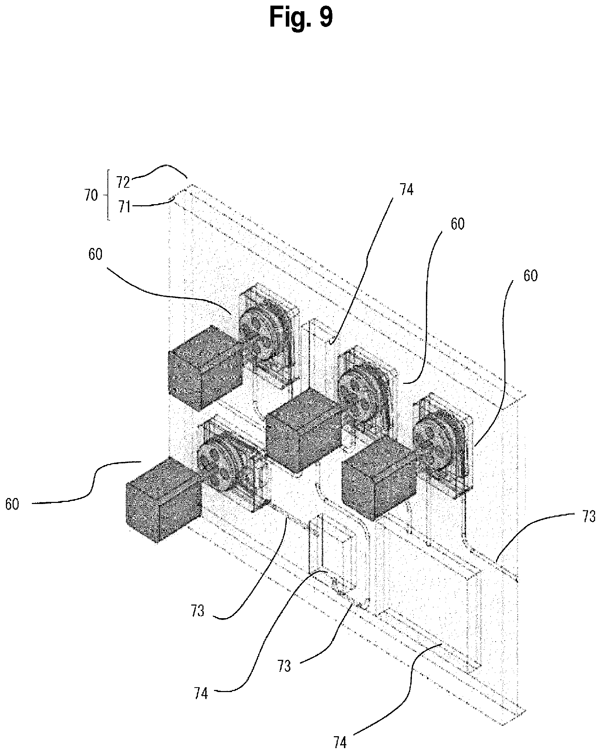

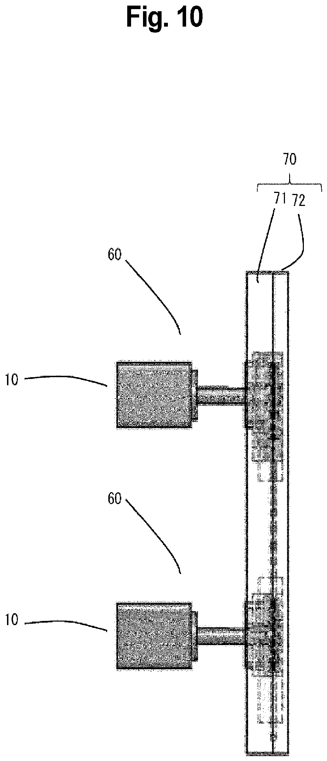

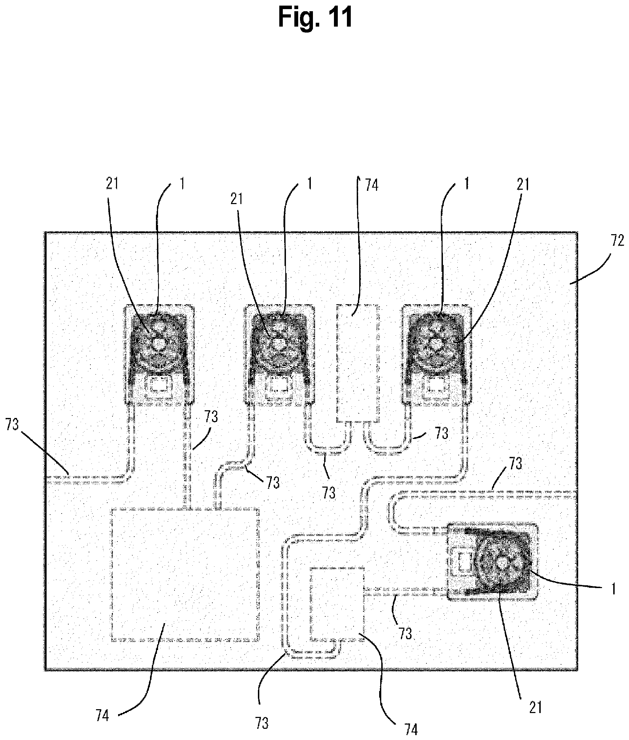

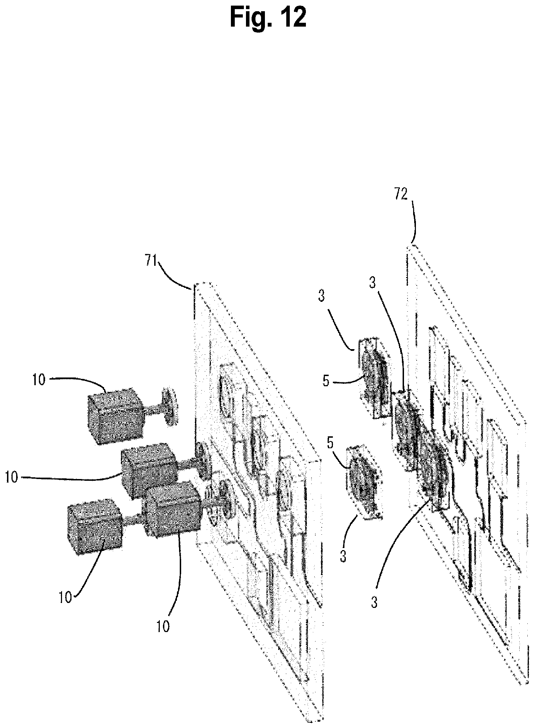

As shown in Figs. 9 to 12, the tube 1 and the tube rotor 21 of the pump 60 may be located in the substrate 70. In addition, the case 3 housing the tube 1 and the tube rotor 21 of the pump 60 may be located in the substrate 70. Alternatively, the case 3 may be omitted. Within the substrate 70, at least a portion of the tube 1 is disposed so as to form a circle-shape portion. For example, a depression that houses or accommodates the tube 1, the tube rotor 21, and the transmission rotor 5 may be disposed on the inner side within the substrate 70. A driver 10, which is positioned on the outside of the substrate 70, may rotate the tube rotor 21 within the substrate 70 by magnetic force. The other constituent elements of the solution transfer device according to the second embodiment, for example, are the same as or similar to those in the solution transfer device according to the first embodiment.

### (Third Embodiment)

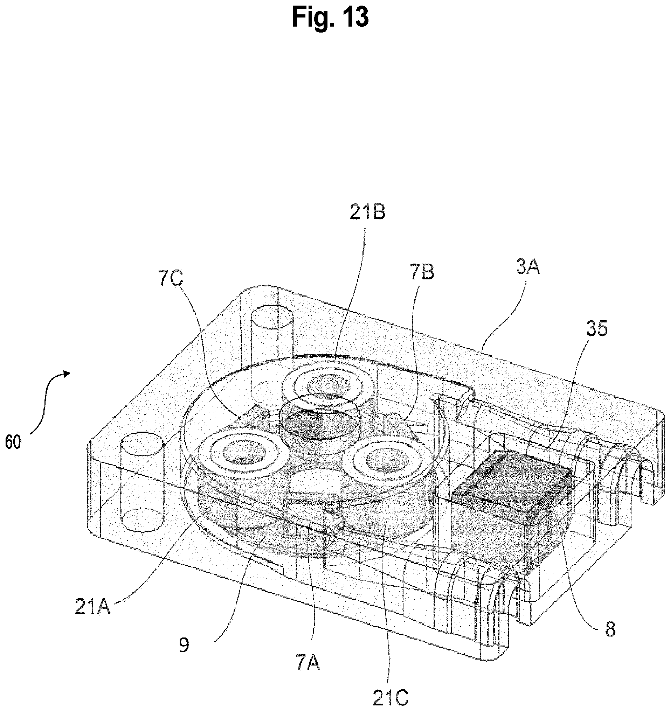

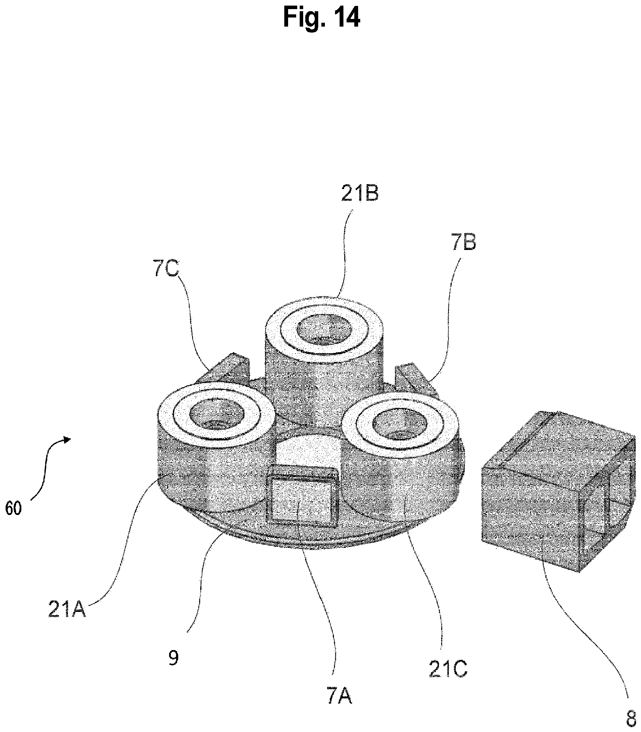

As shown in Figs. 13 and 14, the pump 60 may comprise light reflectors 7A, 7B, 7C, which are connected to at least a portion of the tube rotors 21A, 21B, 21C. The light reflectors 7A, 7B, 7C reflect light. The light reflectors 7A, 7B, 7C are arranged, for example, at equal intervals over the circumference. The light reflectors 7A, 7B, 7C may be disposed on a tube rotor holder member 9 that holds the tube rotors 21A, 21B, 21C. The tube rotor holder member 9 can rotate in conformity with the rotation of the tube rotors 21A, 21B, 21C. In addition, the pump 60 may comprise a light source that irradiates light onto the light reflectors 7A, 7B, 7C from outside the case 3, and with a light-receiver that receives the reflected light from the light reflectors 7A, 7B, 7C. The light-receiver converts the incident reflected light into an electrical signal. The light source and light-receiver may be incorporated in a photoreflector 8.

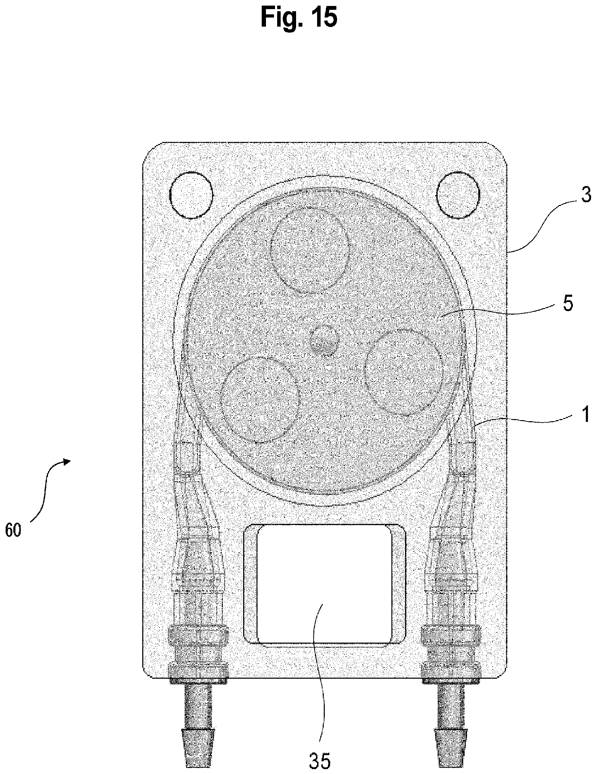

As shown in Figs. 13 and 15, the outer wall of a case may be provided with a housing 35 that houses the light source and the light-receiver. The housing 35 may be a depression or may be a through hole. The light source may directly irradiate the light reflectors 7A, 7B, 7C with light or may irradiate the light reflectors 7A, 7B, 7C with light through an interposed reflector. A reflector that causes refraction of the light from the light source may be provided in the housing 35. The light-receiver may directly receive the reflected light from the light reflectors 7A, 7B, 7C or may receive the reflected light from the light reflectors 7A, 7B, 7C through an interposed reflector. A reflector that causes refraction of the reflected light may be provided in the housing 35.

In the case where the tube rotors 21A, 21B, 21C rotate with respect to the center of the circle-shape portion formed by the tube 1, the light reflectors 7A, 7B, 7C connected to the tube rotors 21A, 21B, 21C also rotate. In the case where light is generated from the light source, the light is reflected when the light reflector 7A, 7B, 7C comes onto the optical axis of the light. The electrical signal generated when the light-receiver receives reflected light assumes a pulse shape. The time interval on which the light-receiver receives light reflected from the light reflectors 7A, 7B, 7C depends on the rotation rate of the tube rotors 21A, 21B, 21C rotating with respect to the center of the circle-shape portion made by the tube 1.

The pump 60 may further comprise a rotation rate calculator that calculates, based on the reflected light received by the light-receiver, the rotation rate of the tube rotors 21A, 21B, 21C rotating with respect to the center of the circle-shape portion made by the tube 1. For example, based on the distance between the light reflectors 7A, 7B, 7C and the length of time for the interval at which the light-receiver receives reflected light, the rotation rate calculator calculates the rotation rate of the tube rotors 21A, 21B, 21C rotating with respect to the center of the circle-shape portion made by the tube 1. The rotation rate calculator, for example, may be incorporated in a computer.

The other constituent elements of the pump 60 according to the third embodiment are the same as or similar to those in the pump 60 according to the first embodiment, and their description is therefore omitted.

In the case where the tube rotors 21A, 21B, 21C are contactlessly rotated by the driver 10, the rotation rate of the drive shaft 11 of the driver 10 may not match the rotation rate of the tube rotors 21A, 21B, 21C rotating with respect to the center of the circle-shape portion made by the tube 1. Due to this, even with monitoring of the rotation rate of the drive shaft 11 of the driver 10, the rotation rate of the tube rotors 21A, 21B, 21C may be different and it may not be possible to advance a fluid within the tube 1 at a desired flow rate. In contrast to this, the pump 60 according to the third embodiment makes it possible to accurately detect the rotation rate of the tube rotors 21A, 21B, 21C, and as a consequence makes it possible to advance the solution within the tube 1 at a desired flow rate.

### (Fourth Embodiment)

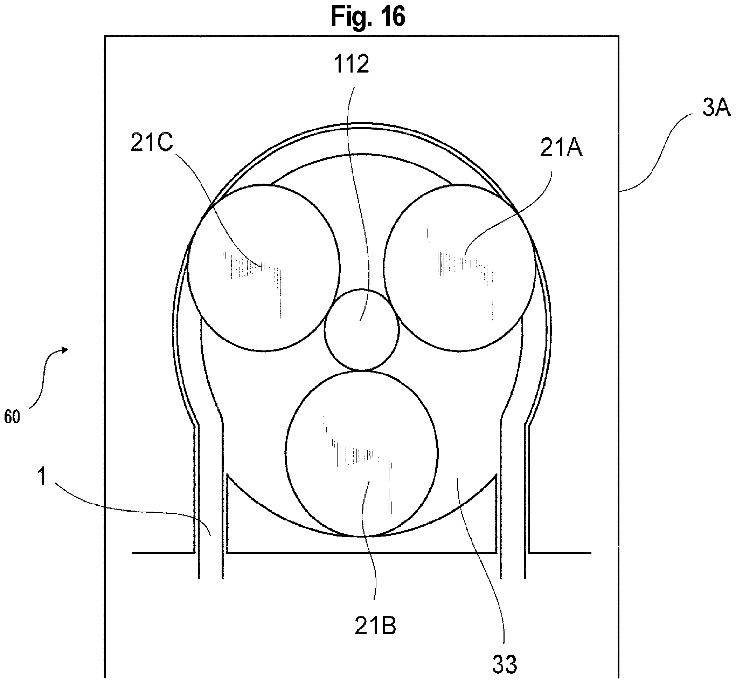

As shown in Fig. 16, the pump 60 may comprise the tube 1 that is disposed along at least a portion of the side surface of a circle-shape depression 33; the tube rotors 21A, 21B, 21C disposed so as to be contactable with the tube 1 within the depression 33; and an internal drive rotor 112 that is in contact with the tube rotors 21A, 21B, 21C. The internal drive rotor 112, for example, has the shape of a shaft. In the case where the internal drive rotor 112 rotates in the pump 60, the tube rotors 21A, 21B, 21C rotate due to frictional force between the internal drive rotor 112 and each of the tube rotors 21A, 21B, 21C.

The pump 60 may comprise a case 3A that is itself provided with the circle-shape depression 33. In this case, the tube 1 is located along at least a portion of the side surface of the depression 33 of the case 3A. In addition, the tube rotors 21A, 21B, 21C are disposed so as to be contactable with the tube 1 within the depression 33 of the case 3A.

Alternatively, a circle-shape depression 33 may be disposed in the interior of the substrate 70 in which the flow path 73 is disposed. In this case, the tube 1 is disposed along at least a portion of the side surface of the depression 33 in the interior of the substrate 70. In addition, the tube rotors 21A, 21B, 21C are disposed so as to be contactable with the tube 1 within the depression 33 in the interior of the substrate 70.

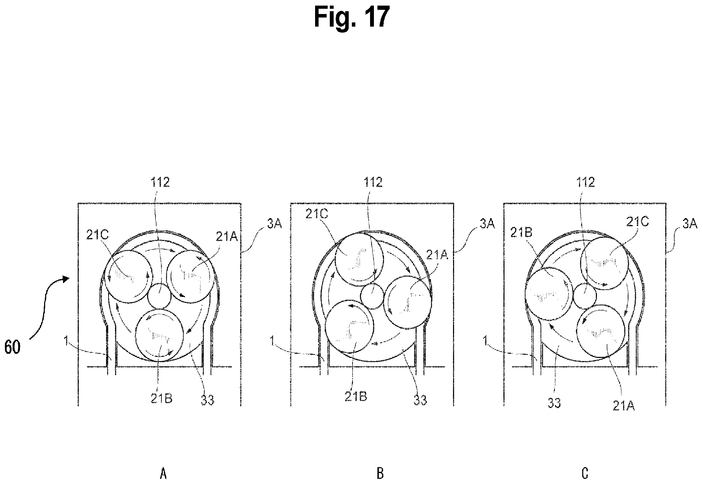

The tube 1 is constituted such that a fluid flows in the interior. The tube rotors 21A, 21B, 21C each have, for example, a cylindrical shape. The number of tube rotors may be freely selected. The outer circumference of each of the tube rotors 21A, 21B, 21C is in contact with the inner circumference of the circle-shape tube 1 and compresses the tube 1. The internal drive rotor 112 is positioned at the center of the circle-shape depression 33. As shown in Fig. 17, in the case where the internal drive rotor 112 rotates in a certain direction, the tube rotor 21A rotates around the center of the tube rotor 21A in the direction opposite from the direction of rotation of the internal drive rotor 112, and at the same time the tube rotor 21A rotates within the depression 33 around the internal drive rotor 112, centered on the internal drive rotor 112, in the same direction as the direction of rotation of the internal drive rotor 112. Of the side surface of the depression 33, the tube rotors 21A, 21B, 21C are in contact with the side surface of the depression 33 in the portion of the side surface where the tube 1 is not provided.

The rotation of each of the tube rotors 21A, 21B, 21C while partially compressing the tube 1 results in the transfer of the fluid within the tube 1 according to the direction of rotation - centered on the internal drive rotor 112 - of each of the tube rotors 21A, 21B, 21C. Since the tube rotors 21A, 21B, 21C are not in contact with the interior of the tube 1, the solution within the tube 1 can be transferred within the tube 1 without contacting the tube rotors 21A, 21B, 21C.

In order to secure frictional force between the internal drive rotor 112 and each of the tube rotors 21A, 21B, 21C, roughness may be provided, e.g., by serration machining, in the surface of the internal drive rotor 112 that comes into contact with each of the tube rotors 21A, 21B, 21C.

The radius of each of the tube rotors 21A, 21B, 21C may be longer than the radius of the internal drive rotor 112. By making the radius of each of the tube rotors 21A, 21B, 21C longer than the radius of the internal drive rotor 112, the rotation rate of each of the tube rotors 21A, 21B, 21C is made slower that the rotation rate of the internal drive rotor 112. By doing this, each of the tube rotors 21A, 21B, 21C can provide a larger torque than the torque of the internal drive rotor 112.

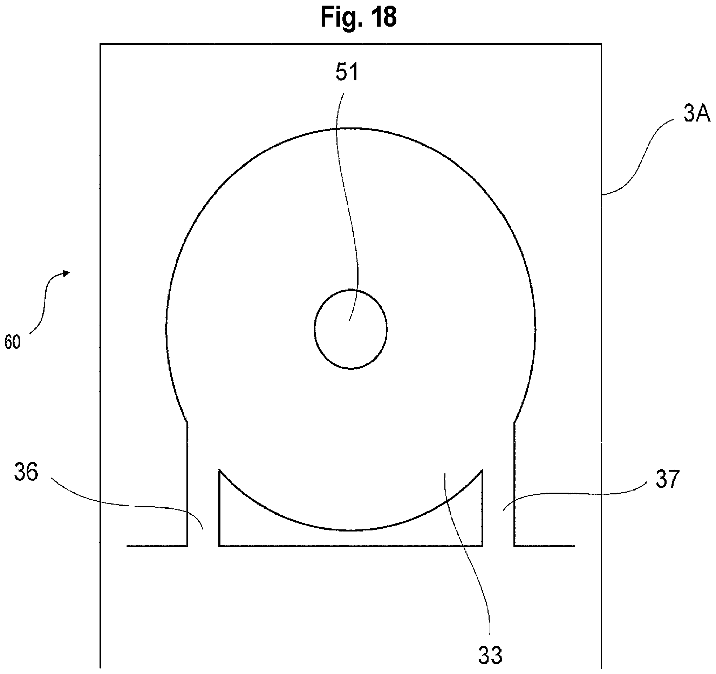

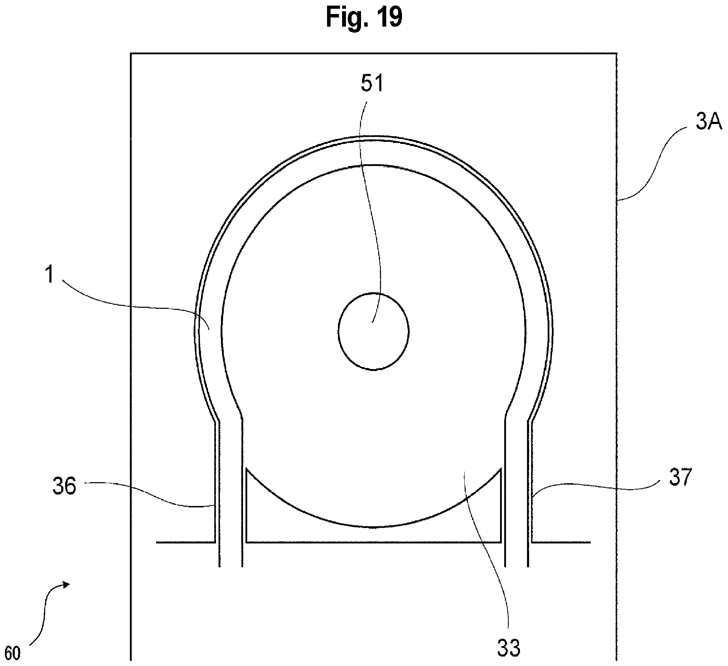

As shown in Fig. 18, a depression 36, which is provided at one end side of the tube 1, and a depression 37, which is provided at the other end side of the tube 1, may be connected to the circle-shape depression 33. In addition, a bearing 51 for the internal drive rotor 112 may be provided in the circle-shape depression 33. As shown in Fig. 19, the tube 1 is disposed so as to run within the depression 36 and the depression 37 and along the side surface of the circle-shape depression 33 between the depression 36 and the depression 37. After this, the internal drive rotor 112 shown in Fig. 16 may be placed in the bearing 51 and the tube rotors 21A, 21B, 21C may be inserted between the internal drive rotor 112 and the tube 1 and between the internal drive rotor 112 and the side surface of the depression 33 where the tube 1 is not provided.

In the case where the interior of the case 3A is to be closed, the case 3B and the transmission rotor 5 shown in

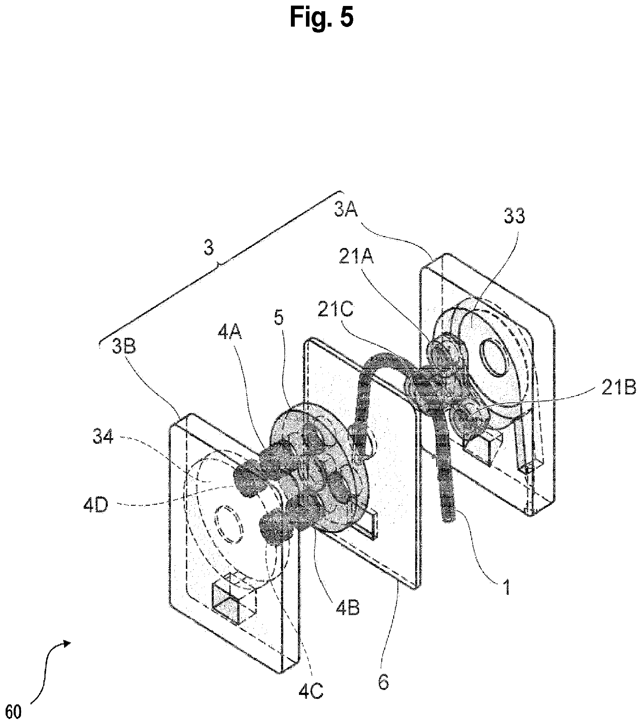

Fig. 5 may be prepared and the internal drive rotor 112 may be connected to the center of the transmission rotor 5. By doing this, the driver 10 can rotate, from outside the case 3, the interior tube rotors 21A, 21B, 21C closed off by the case 3A and the case 3B. In this manner, the structure of the pump according to the fourth embodiment may be combined with the structure of the pump according to the first embodiment.

### (Fifth Embodiment)

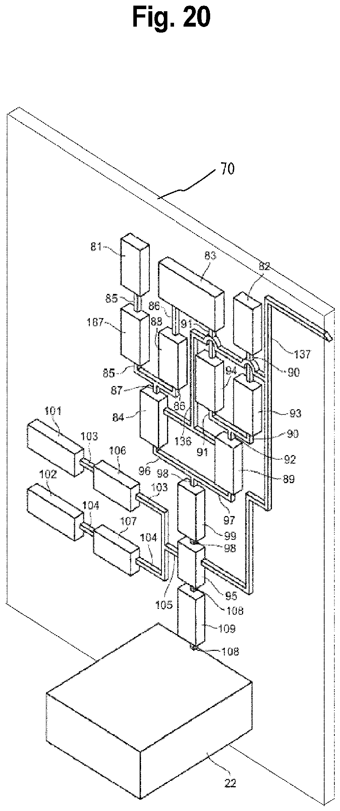

The cell treatment section 22 may be disposed outside the substrate 70, as shown in Fig. 20. For example, the cell treatment section 22 may project out from a side surface of the substrate 70. The cell treatment section 22 may project out perpendicularly from a side surface of the substrate 70. By doing this, the cell treatment section 22 will be horizontal in the case where the substrate 70 is disposed perpendicular to a flat surface. As a consequence, for example, the adhesion culture of cells within the cell treatment section 22 can be facilitated. For example, cells into which a factor is to be introduced are transported into the cell treatment section 22 through a pump and a flow path that are not shown.

A factor container 81 that stores a factor, and a reagent container 82 that stores a reagent for introducing the factor into cells, may be connected to the cell treatment section 22 through flow paths provided in the substrate 70. The factor container 81 provided in the substrate 70 stores in the former's interior a factor to be introduced into cells.

The reagent container 82 provided in the substrate 70 stores a reagent for introducing the factor stored in the factor container 81 into cells. This reagent can be exemplified by artificial liposomes, cationic lipids, calcium chloride, cationic diethylaminoethyldextran molecules, cationic peptides and derivatives thereof, straight-chain or branched-chain synthetic polymers, polysaccharide-based transduction molecules, natural polymers, and active dendrimers and inactive dendrimers. The reagent is, for example, a transfection reagent. In the present disclosure, transfection refers to the introduction into cells not only of nucleic acid, but also protein, compounds, and viruses.

A diluent container 83 for storing a diluent for diluting each of the factor and reagent may be provided in the substrate 70. The diluent can be exemplified by phosphate-buffered physiological saline (PBS).

The substrate 70 may be provided with a factor dilution container 84 for mixing the diluent with the factor, and which is disposed in a flow path between the cell treatment section 22 and the factor container 81 and the diluent container 83. Factor that comes from the factor container 81 and diluent that comes from the diluent container 83 are mixed in the factor dilution container 84 to produce a dilution of the factor. The factor dilution container 84 may be a mixer that is provided with a bent flow path through which the dilution of the factor flows. The bent flow path may be bent in a helical or spiral shape. The flow path in the bent flow path may have a meandering course. The cross-sectional area in the bent flow path may repeatedly increase and decrease.

A flow path 85 is connected to the factor container 81 for transporting at least a factor from the factor container 81 to the factor dilution container 84. A flow path 86 is connected to the diluent container 83 for transporting at least diluent from the diluent container 83 to the factor dilution container 84. The flow path 85 and the flow path 86 combine into a flow path 87. The flow path 87 is connected to the factor dilution container 84. A pump 187 for transferring the fluid within the flow path 85 may be provided in the flow path 85. A valve other than a pump may not be provided in the flow path 85. A pump 88 for transferring the fluid within the flow path 86 may be provided in the flow path 86. A valve other than a pump may not be provided in the flow path 86. The pump used in the fifth embodiment may be the same as or similar to the pumps described in any of the first to the fourth embodiments.

The pump 187 transfers the factor in the factor container 81 through the flow paths 85, 87 into the factor dilution container 84. In addition, the pump 88 transfers the diluent in the diluent container 83 through the flow paths 86, 87 into the factor dilution container 84. The pump 187 may carry out a quantitative transfer of the factor in the factor container 81 into the factor dilution container 84. The pump 88 may carry out a quantitative transfer of the diluent in the diluent container 83 into the factor dilution container 84. Otherwise, the pumps may not be provided in the flow paths 85, 86 and a pump may be provided in the flow path 87, and the pump provided in the flow path 87 may transfer the factor in the factor container 81 and the diluent in the diluent container 83 into the factor dilution container 84.

The factor dilution container 84, for example, may be connected, through flow paths 135, 136, 137 and a flow path 131, to a storage tank that is not shown. In the case where the factor and diluent are transferred into the factor dilution container 84, fluid, e.g., gases, e.g., air, within the factor dilution container 84, for example, are transferred into the storage tank that is not shown.

A reagent dilution container 89 may be provided in the substrate 70 for mixing a reagent and a diluent, and which is provided in a flow path between the cell treatment section 22 and the reagent container 82 and the diluent container 83. Reagent that comes from the reagent container 82 and diluent that comes from the diluent container 83 are mixed in the reagent dilution container 89 to prepare a dilution of the reagent. The reagent dilution container 89 may be a mixer that is provided with a bent flow path through which the dilution of the reagent flows. The bent flow path may be bent in a helical or spiral shape. The flow path in the bent flow path may have a meandering course. The cross-sectional area in the bent flow path may repeatedly increase and decrease.

A flow path 90 for transferring at least reagent from the reagent container 82 to the reagent dilution container 89 is connected to the reagent container 82. A flow path 91 for transferring at least diluent from the diluent container 83 to the reagent dilution container 89 is connected to the diluent container 83. The flow path 90 and the flow path 91 combine into a flow path 92. The flow path 92 is connected to the reagent dilution container 89. A pump 93 for transporting the fluid in the flow path 90 may be provided in the flow path 90. A valve other than a pump may not be provided in the flow path 90. A pump 94 for transporting the fluid in the flow path 91 may be provided in the flow path 91. A valve other than a pump may not be provided in the flow path 91.

The pump 93 transfers the reagent in the reagent container 82, through the flow paths 90, 92, into the reagent dilution container 89. In addition, the pump 94 transfers the diluent in the diluent container 83, through the flow paths 91, 92, into the reagent dilution container 89. The pump 93 may quantitatively transfer the reagent in the reagent container 82 into the reagent dilution container 89. The pump 94 may quantitatively transfer the diluent in the diluent container 83 into the reagent dilution container 89. Otherwise, the pumps may not be provided in the flow paths 90, 91 and a pump may be provided in the flow path 92, and the pump provided in the flow path 92 may transfer the reagent in the reagent container 82 and the diluent in the diluent container 83 into the reagent dilution container 89.

The reagent dilution container 89, for example, may be connected, through flow paths 135, 136, 137 and a flow path 131, to a storage tank that is not shown. In the case where the reagent and diluent are transferred into the reagent dilution container 89, fluid, e.g., gases, e.g., air, within the reagent dilution container 89, for example, are transferred into the storage tank that is not shown.

A mixing tank 95 for mixing the factor and reagent, and provided in a flow path between the cell treatment section 22 and the factor dilution container 84 and reagent dilution container 89, may be provided in the substrate 70. A factor/reagent mixed solution is prepared by mixing, in the mixing tank 95, the dilution of the factor that comes from the factor dilution container 84 and the dilution of the reagent that comes from the reagent dilution container 89. The mixing tank 95 may be a mixer that is provided with a bent flow path through which the factor/reagent mixed solution flows. The bent flow path may be bent in a helical or spiral shape. The flow path in the bent flow path may have a meandering course. The cross-sectional area in the bent flow path may repeatedly increase and decrease.

A flow path 96 is connected to the factor dilution container 84 for transporting at least a dilution of the factor from the factor dilution container 84 to the mixing tank 95. A flow path 97 is connected to the reagent dilution container 89 for transporting at least a dilution of the reagent from the reagent dilution container 89 to the mixing tank 95. The flow path 96 and the flow path 97 combine into a flow path 98. The flow path 98 is connected to the mixing tank 95. A pump 99 for transferring the fluid within the flow path 98 may be provided in the flow path 98. A valve other than a pump may not be provided in the flow path 98.

The pump 99 transfers the dilution of the factor in the factor dilution container 84 to the mixing tank 95 through the flow paths 96, 98. In addition, the pump 99 transfers the dilution of the reagent in the reagent dilution container 89 into the mixing tank 95 through the flow paths 97, 98. The pump 99 may quantitatively transfer the dilution of the factor in the factor dilution container 84 into the mixing tank 95. The pump 99 may quantitatively transfer the dilution of the reagent in the reagent dilution container 89 into the mixing tank 95.

The mixing tank 95, for example, may be connected, through the flow path 137 and the flow path 131, to a storage tank that is not shown. In the case where the dilution of the factor and the dilution of the reagent are transferred into the mixing tank 95, fluid, e.g., gases, e.g., air, within the mixing tank 95, for example, are transferred into the storage tank that is not shown.

A plurality of culture medium containers 101, 102, each of which contains a culture medium to be supplied to the mixing tank 95, may be connected to the mixing tank 95.

A flow path 103 for transferring at least a culture medium from the culture medium container 101 to the mixing tank 95 is connected to the culture medium container 101. A flow path 104 for transferring at least a culture medium from the culture medium container 102 to the mixing tank 95 is connected to the culture medium container 102. The flow path 103 and the flow path 104 combine into a flow path 105. The flow path 105 is connected to the mixing tank 95. A pump 106 for transferring the fluid in the flow path 103 may be provided in the flow path 103. A valve other than a pump may not be provided in the flow path 103. A pump 107 for transferring the fluid in the flow path 104 may be provided in the flow path 104. A valve other than a pump may not be provided in the flow path 104.

The pump 106 transfers the culture medium in the culture medium container 101 into the mixing tank 95 through the flow paths 103, 105. In addition, the pump 107 transfers the culture medium in the culture medium container 102 into the mixing tank 95 through the flow paths 104, 105. The pump 106 may quantitatively transfer the culture medium in the culture medium container 101 into the mixing tank 95. The pump 107 may quantitatively transfer the dilution of the reagent in the culture medium container 102 into the mixing tank 95.

The factor is mixed with the reagent and culture medium in the mixing tank 95. The factor and reagent may be used in microamounts; however, the volume is increased by the admixture of the culture medium and feed to the cell treatment section 22 may then be facilitated.

The plurality of the culture medium containers 101, 102 may store different culture media. For example, a different culture medium may be transported into the mixing tank 95 from either of the plurality of the culture medium containers 101, 102, in correspondence to the number of times of transport of the reagent and factor from the mixing tank 95 to the cell treatment section 22. In addition, for example, a different culture medium may be transported to the mixing tank 95 from either of the plurality of the culture medium containers 101, 102, in correspondence to the timing of the transport of the reagent and factor from the mixing tank 95 into the cell treatment section 22. For example, in the case where a factor is introduced into cells in the cell treatment section 22 to cause the cells to change from a first state to a second state, a culture medium adapted to the cells in the first state may be supplied, in the initial stage of factor introduction, to the mixing tank 95 from the culture medium container 101, while, in a later stage of factor introduction, a culture medium adapted to the cells in the second state may be supplied to the mixing tank 95 from the culture medium container 102.

A flow path 108 for transporting factor and reagent from the mixing tank 95 to the cell treatment section 22 is connected to the mixing tank 95. A pump 109 for transferring the fluid in the flow path 108 may be provided in the flow path 108.

The pump 109 transfers the factor and reagent in the mixing tank 95 through the flow path 108 into the cell treatment section 22. The pump 109 may quantitatively transfer the factor and reagent in the mixing tank 95 into the cell treatment section 22. The pump 109 may transport the reagent and factor from the mixing tank 95 to the cell treatment section 22 a prescribed number of times. In addition, the pump 109 may transport the reagent and factor from the mixing tank 95 to the cell treatment section 22 according to a prescribed timing.

The cells in the cell treatment section 22 are brought into contact with the factor and reagent and the factor is thereby introduced into the cells. The factor is quantitatively introduced into the cells by the quantitative transfer of the factor and reagent in the mixing tank 95 into the cell treatment section 22. The factor is introduced into the cells a prescribed number of times by transferring the factor and reagent in the mixing tank 95 into the cell treatment section 22 a prescribed number of times. The factor is introduced into the cells according to a prescribed timing by transferring the factor and reagent in the mixing tank 95 into the cell treatment section 22 according to a prescribed timing.

### (Sixth Embodiment)

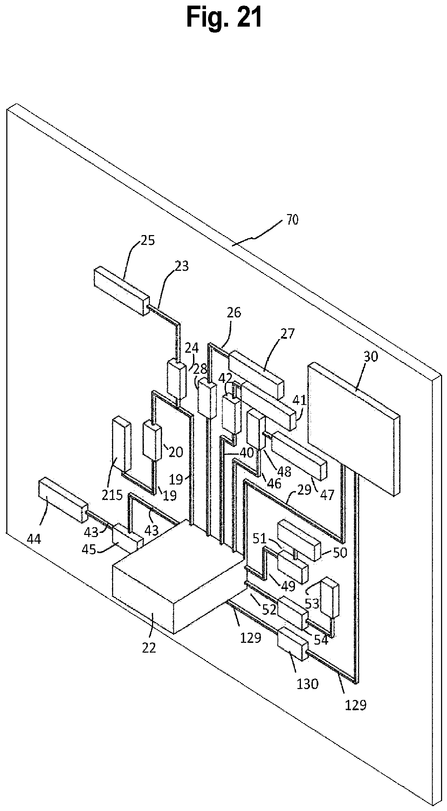

According to the example shown in Fig. 21, the cell treatment section 22 is also provided outside of the substrate 70. For example, the cells into which a factor is to be introduced are transported into the cell treatment section 22, through a flow path 19 and a pump 20 provided in the substrate 70, from a cell container 215 provided in the substrate 70. The pump used in the sixth embodiment is the same as or similar to the pumps described in any of the first to fourth embodiments.

A flow path 23 is connected to the flow path 19. A pump 24 for transferring the fluid in the flow path 23 is provided in the flow path 23 that is provided in the substrate 70. A valve other than a pump may not be provided in the flow path 23.

There is connected to the flow path 23, for example, a first culture medium container 25, which is provided in the substrate 70 and is a fluid container that holds a somatic cell culture medium, e.g., a differentiated cell culture medium, or holds a stem cell culture medium adapted to, e.g., iPS cells, ES cells, stem cells, and so forth. The culture medium may be a gel, a liquid, or a flowable solid. Flowable solids can be exemplified by agar and temperature-sensitive gels.

In the case where the culture medium is a gel, the culture medium may contain a polymer compound. This polymer compound may be, for example, at least one selection from the group consisting of gellan gum, deacylated gellan gum, hyaluronic acid, rhamsan gum, diutan gum, xanthan gum, carrageenan, fucoidan, pectin, pectic acid, pectinic acid, heparan sulfate, heparin, heparitin sulfate, keratosulfate, chondroitin sulfate, dermatan sulfate, rhamnan sulfate, and salts of the preceding. The culture medium may also contain methyl cellulose. Aggregation among cells is better inhibited by the incorporation of methyl cellulose.

Alternatively, the culture medium may contain a small amount of a temperature-sensitive gel selected from poly(glycerol monomethacrylate) (PGMA); poly(2-hydroxypropyl methacrylate) (PHPMA); poly(N-isopropylacrylamide) (PNIPAM); and amine-terminated, carboxylic acid-terminated, maleimide-terminated, N-hydroxysuccinimide (NHS) ester-terminated, or triethoxysilane-terminated poly(N-isopropylacrylamide-co-acrylamide), poly(N-isopropylacrylamide-co-acrylic acid), poly(N-isopropylacrylamide-co-butyl acrylate), poly(N-isopropylacrylamide-co-methacrylic acid), poly(N-isopropylacrylamide-co-methacrylic acid-co-octadecyl acrylate), and N-isopropylacrylamide. In the present disclosure, gel-form culture medium or gel culture medium encompasses polymer culture media.

In the case where the cells transported from the flow path 19 into the cell treatment section 22 are mononuclear cells, which are somatic cells, for example, a blood cell culture medium can be used as a somatic cell culture medium. In the case where, for example, mononuclear cells are transported into the flow path 19, the pump 24 transports a somatic cell culture medium from the first culture medium container 25 to the flow path 19 through the flow path 23. The somatic cell culture medium that has been transported into the flow path 19 through the flow path 23 and the mononuclear cells in the flow path 19 are mixed and are transported into the cell treatment section 22. Preliminarily prepared mononuclear cells may be supplied into the cell treatment section 22. The cells transported to the cell treatment section 22 are not limited to mononuclear cells, and may be any cells, e.g., somatic cells and so forth.

At least either of the first culture medium container 25 and the flow path 23 may be provided with a temperature regulator that regulates the temperature of the culture medium in the first culture medium container 25. Even after cells have been transported into the cell treatment section 22, the pump 24 may transport somatic cell culture medium into the cell treatment section 22 from the first culture medium container 25.

For example, a container 30 provided in the substrate 70 is connected to the cell treatment section 22 through a flow path 29 provided in the substrate 70. A pump for transferring the fluid within the flow path 29 may be provided in the flow path 29. A valve other than a pump may not be provided in the flow path 29. In the case where somatic cells and somatic cell culture medium are transported from the flow path 19 into the cell treatment section 22, gas, e.g., air, in the cell treatment section 22, for example, is transferred into the container 30.

For example, a container 27 provided in the substrate 70 is connected to the cell treatment section 22 through a flow path 26 provided in the substrate 70. A pump 28 for transferring the fluid within the flow path 26 may be provided in the flow path 26. The container 27, for example, stores a factor in its interior. In the case where iPS cells are to be produced by the introduction of a factor, e.g., a reprogramming factor, into somatic cells, the pump 28 transfers a somatic cell-containing somatic cell culture medium in the cell treatment section 22 through the flow path 26 into the container 27. The somatic cells, through their transfer from within the cell treatment section 22 into the container 27, come into contact with the factor in the container 27 and the factor is thereby introduced into the somatic cells.

For example, a coating agent container 44 provided in the substrate 70, this being a fluid container that stores a cell adhesion coating agent, e.g., Matrigel, collagen, polylysine, fibronectin, vitronectin, gelatin, laminin, and so forth, is connected to the cell treatment section 22 through a flow path 43 provided in the substrate 70. A pump 45 for transferring the fluid in the flow path 43 may be provided in the flow path 43. A valve other than a pump may not be provided in the flow path 43.

During the interval in which the cells are incorporating the factor in the container 27, the pump 45 transfers, through the flow path 43, the cell adhesion coating agent present in the coating agent container 44 into the cell treatment section 22. The bottom surface of the cell treatment section 22 is coated by the cell adhesion coating agent as a consequence. In the case where the cell adhesion coating agent is transported into the cell treatment section 22 from the flow path 43, the excess fluid in the cell treatment section 22, for example, is transferred into the container 30.

A flow path 129 may be provided between the cell treatment section 22 and the container 30. A pump 130 may be provided in the flow path 129. A valve other than a pump may not be provided in the flow path 129. After the bottom surface of the cell treatment section 22 has been coated with the cell adhesion coating agent for a prescribed period of time, the pump 130 transfers the cell adhesion coating agent in the cell treatment section 22 into the container 30 through the flow path 129.

After the cell adhesion coating agent has been transferred into the container 30, the pump 28 transfers the somatic cell culture medium containing factor-transduced somatic cells in the container 27, into the cell treatment section 22 through the flow path 26.

The coating agent container 44 need not be present in the case where the cell adhesion coating agent has been previously coated on the bottom surface of the cell treatment section 22 or in the case where the cell adhesion coating agent has been previously introduced into the cell treatment section 22. Also in this case, the pump 28 may transfer the factor in the container 27 into the cell treatment section 22 through the flow path 26. The factor, through its transfer from the container 27 into the cell treatment section 22, comes into contact with the somatic cells in the cell treatment section 22 and the factor is thereby introduced into the somatic cells. The transfer of the induction factor in the container 27 through the flow path 26 into the cell treatment section 22 by the pump 28 may be divided into a plurality of transfers. Introduction of the induction factor into the somatic cells may be divided into a plurality of introductions as a consequence.

For example, a second culture medium container 41 provided in the substrate - this being a fluid container that stores a culture medium, e.g., a stem cell culture medium, somatic cell culture medium, and so forth - is connected to the cell treatment section 22 through a flow path 40 provided in the substrate 70. An example in which the second culture medium container 41 holds a stem cell culture medium is described in the following. The stem cell culture medium may be a gel, a liquid, or a flowable solid. The stem cell culture medium may contain agar and/or a temperature-sensitive gel. A culture medium for induction culture, or for expansion culture, or for maintenance culture can be used as the stem cell culture medium.

At least either of the second culture medium container 41 and the flow path 40 may be provided with a temperature regulator that regulates the temperature of the culture medium in the second culture medium container 41. A pump 42 for transferring the fluid in the flow path 40 may be provided in the flow path 40. After the passage of a prescribed period of time after the introduction of the induction factor to the somatic cells, the pump 42 transfers the stem cell culture medium in the second culture medium container 41 into the cell treatment section 22 through the flow path 40. In the case where the stem cell culture medium is transported into the cell treatment section 22 from the flow path 40, for example, culture medium and gas, e.g., air, in the cell treatment section 22 is transferred through the flow path 29 into the container 30.

During the period in which the cells are cultured in the cell treatment section 22, the pump 42 may transfer, in accordance with a prescribed timing, the stem cell culture medium in the second culture medium container 41 into the cell treatment section 22 through the flow path 40. In addition, the culture medium may be circulated between the second culture medium container 41 and the cell treatment section 22. The pump 42 may control the amount of culture medium transported, and/or may start and finish culture medium transport, in correspondence to changes in the state of the culture medium, state of the cell clusters in the culture medium, number of cells, number of cell clusters, turbidity of the culture medium, and pH.

For example, a detachment solution container 47 provided in the substrate 70, this being a fluid container that stores a cell dissociation agent, e.g., trypsin, TrypLE Select, Accutase, EDTA, and so forth, is connected to the cell treatment section 22 through a flow path 46 provided in the substrate 70. A pump 48 for transferring the fluid in the flow path 46 may be provided in the flow path 46. A valve other than a pump may not be provided in the flow path 46.

The pump 48 transfers the cell dissociation agent in the detachment solution container 47 through the flow path 46 into the cell treatment section 22. The cells adhered to the bottom surface of the cell treatment section 22 are exposed to the cell dissociation agent as a result. In the case where the cell dissociation agent is transported into the cell treatment section 22 from the flow path 46, the excess fluid in the cell treatment section 22, for example, is transferred into the container 30. At least either of the detachment solution container 47 and the flow path 46 may be provided with a temperature regulator that regulates the temperature of the cell dissociation agent in the detachment solution container 47.

After the cells in the cell treatment section 22 have been exposed to the cell dissociation agent for a prescribed period of time at a prescribed temperature, the pump 130 transfers the cell dissociation agent in the cell treatment section 22 into the container 30 through the flow path 129. After the further passage of a prescribed period of time at a prescribed temperature, the cells are then detached from the bottom surface of the cell treatment section 22. All or a portion of the detached cells in the cell treatment section 22 are transported out to the container 30 through the flow path 129. At least a portion of the cells transported to the outside of the cell treatment section 22 may be returned to the cell treatment section 22. Subsequent to this, stem cell culture medium is supplied into the cell treatment section 22 from the second culture medium container 41. After adhesion of the cells to the bottom surface of the cell treatment section 22 and the further passage of a prescribed period of time, for example, a Sendai virus vector may be extinguished at high temperatures, e.g., 38°C. Factor introduction into cells may be repeated a plurality of times, e.g., two times, three times, and so forth. Subsequent to this the cells in the cell treatment section 22 are detached from the bottom surface of the cell treatment section 22.

For example, a cryopreservation solution container 50 provided in the substrate 70, this being a fluid container that stores a cell cryopreservation solution, is connected to the cell treatment section 22 through a flow path 49. A pump 51 for transferring the fluid in the flow path 49 may be provided in the flow path 49. A valve other than a pump may not be provided in the flow path 49.

For example, after iPS cells have been prepared in the cell treatment section 22 from somatic cells that have incorporated the induction factor, the pump 51 transfers the cell cryopreservation solution present in the cryopreservation solution container 50 through the flow path 49 into the cell treatment section 22. The cells in the cell treatment section 22 are incorporated in the cell cryopreservation solution as a result. In the case where the cell cryopreservation solution is transported into the cell treatment section 22 from the flow path 49, the excess fluid in the cell treatment section 22, for example, is transferred to the container 30.

For example, a cell cryopreservation container 53, which is a fluid container that stores a cell cryopreservation solution, is connected to the cell treatment section 22 through a flow path 52 provided in the substrate 70. A pump 54 for transferring the fluid in the flow path 52 may be provided in the flow path 52. The pump 54 transports the cell-containing cell cryopreservation solution in the cell treatment section 22 to the cell cryopreservation container 53. A valve other than a pump may not be provided in the flow path 52.

The present invention has been described in the preceding using embodiments, but the description and figures that comprise a part of this disclosure should not be understood as being limitations on this invention. It can be expected that various alternative embodiments, embodiments, and operating art will become clear to the individual skilled in the art based on this disclosure. It should be understood that the present invention encompasses various embodiments and so forth that are not described herein.

### Reference Signs List

- 1: Tube
- 3, 3A, 3B: Case
- 4A, 4B, 4C, 4D: Magnet
- 5: Transmission rotor
- 6: Shaft holder
- 7A, 7B, 7C: Light reflector
- 8: Photoreflector
- 9: Tube rotor holder member
- 10: Driver
- 11: Drive shaft
- 12: External drive rotor
- 13A, 13B, 13C, 13D: Magnet
- 19: Flow path
- 20: Pump
- 21, 21A, 21B, 21C: Tube rotor
- 22: Cell treatment section
- 23: Flow path
- 24: Pump
- 25: Culture medium container
- 26: Flow path
- 27: Container
- 28: Pump
- 29: Flow path
- 30: Container
- 31: Inlet connector
- 32: Outlet connector
- 33, 34, 36, 37: Depression
- 35: Housing
- 40: Flow path
- 41: Culture medium container
- 42: Pump
- 43: Flow path
- 44: Coating agent container
- 45: Pump
- 46: Flow path
- 47: Detachment solution container
- 48: Pump
- 49: Flow path
- 50: Cryopreservation solution container
- 51: Pump
- 52: Flow path
- 53: Cell cryopreservation container
- 54: Pump
- 60: Pump
- 70: Substrate
- 71: First substrate section
- 72: Second substrate section
- 73: Flow path
- 74: Cell treatment section
- 75: First contact surface
- 76: Second contact surface
- 77: Outside surface
- 78: Hole
- 81: Factor container
- 82: Reagent container
- 83: Diluent container
- 84: Factor dilution container
- 85, 86, 87: Flow path
- 88: Pump
- 89: Reagent dilution container
- 90, 91, 92: Flow path
- 93, 94: Pump
- 95: Mixing tank
- 96, 97, 98: Flow path
- 99: Pump
- 101, 102: Culture medium container
- 103, 104, 105: Flow path
- 106, 107: Pump
- 108: Flow path
- 109: Pump
- 112: Internal drive rotor
- 129: Flow path
- 130: Pump
- 131, 135, 137: Flow path
- 187: Pump
- 215: Cell container

## Claims

1. A solution transfer device comprising
a pump comprising a tube for transferring a solution, a tube rotor that contacts the tube, and a driver that rotates the tube rotor without contacting the tube rotor, in order to transfer the solution within the tube; and
a substrate provided with a flow path for transferring the solution, wherein the flow path is connected to the tube of the pump.

2. The solution transfer device according to claim 1, wherein the flow path is provided in the interior of the substrate.

3. The solution transfer device according to claim 1 or 2, wherein the solution is a solution containing a cell.

4. The solution transfer device according to any one of claims 1 to 3, wherein the solution is a solution containing a reagent.

5. The solution transfer device according to any one of claims 1 to 4, wherein the solution is a solution containing a culture medium.

6. The solution transfer device according to claim 3, wherein a cell treatment section for treating the cells is provided at the substrate and is connected to the flow path.

7. The solution transfer device according to claim 6, wherein the cell treatment section is a purification section for purifying the cells.

8. The solution transfer device according to claim 6, wherein the cell treatment section is a factor introduction section for introducing a factor to the cells.

9. The solution transfer device according to claim 6, wherein the cell treatment section is a culture section for culturing the cells.

10. The solution transfer device according to claim 6, wherein the cell treatment section is a differentiation induction section for inducing the differentiation of the cells.

11. The solution transfer device according to any one of claims 1 to 10, wherein the tube and the tube rotor of the pump are disposed outside of the substrate.

12. The solution transfer device according to any one of claims 1 to 11, wherein the tube and the tube rotor of the pump are disposed in the substrate.

13. The solution transfer device according to any one of claims 1 to 12, wherein the driver is disposed outside of the substrate.

14. The solution transfer device according to any one of claims 1 to 13, wherein
the pump further comprises a magnet that is connected to the tube rotor; and
the driver causes rotation of the tube rotor by magnetic force using the magnet.

15. The solution transfer device according to any one of claims 1 to 14, wherein
the substrate is provided with a first substrate section having a first contact surface and with a second substrate section having a second contact surface that contacts the first contact surface; and
the flow path is provided in at least one of the first contact surface and the second contact surface.

16. The solution transfer device according to any one of claims 1 to 15, wherein the pump further comprises a case that houses the tube and the tube rotor.

17. The solution transfer device according to claim 16, wherein the driver rotates the tube rotor, from outside the case, without contacting the tube rotor.

18. A solution transfer device comprising
a pump that comprises a tube for transferring solution disposed along at least a portion of a side surface of a circle-shape depression, a tube rotor disposed to be contactable with the tube at within the depression, and an internal drive rotor that contacts the tube rotor, wherein, in the case where the internal drive rotor rotates, the tube rotor is rotated by frictional force between the internal drive rotor and the tube rotor; and
a substrate provided with a flow path for transferring the solution, wherein the flow path is connected to the tube of the pump.

19. The solution transfer device according to claim 18, wherein the flow path is provided in the interior of the substrate.

20. The solution transfer device according to claim 18 or 19, wherein the solution is a cell-containing solution.

21. The solution transfer device according to claim 20, wherein a cell treatment section for treating the cells is provided at the substrate and is connected to the flow path.

22. A solution transfer device according to claim 21, wherein the cell treatment section is a purification section for purifying the cells.

23. The solution transfer device according to claim 21, wherein the cell treatment section is a factor introduction section for introducing a factor to the cells.

24. The solution transfer device according to claim 21, wherein the cell treatment section is a culture section for culturing the cells.

25. The solution transfer device according to claim 21, wherein the cell treatment section is a differentiation induction section for inducing the differentiation of the cells.

26. A solution transfer device according to any one of claims 18 to 25, wherein the depression is provided in the substrate.

27. The solution transfer device according to any one of claims 18 to 25, wherein the pump further comprises a case in which the depression is provided.

28. The solution transfer device according to any one of claims 18 to 27, wherein the pump further comprises a driver for transferring the solution in the tube by rotating the internal drive rotor without contacting the internal drive rotor.

29. The solution transfer device according to any one of claims 18 to 28, wherein
the substrate is provided with a first substrate section having a first contact surface and with a second substrate section having a second contact surface that contacts the first contact surface, and
the flow path is provided in at least one of the first contact surface and the second contact surface.

30. The solution transfer device according to any one of claims 21 to 25, wherein
the substrate is provided with a first substrate section having a first contact surface and with a second substrate section having a second contact surface that contacts the first contact surface, and
the cell treatment section is provided in at least one of the first contact surface and the second contact surface.

31. The solution transfer device according to any one of claims 18 to 30, wherein the tube rotor rotates around the center of the tube rotor and rotates within the depression centered on the internal drive rotor.

32. The solution transfer device according to any one of claims 18 to 31, wherein the radius of the tube rotor is longer than the radius of the internal drive rotor.
